# EUROPEAN PATENT APPLICATION

(11) **EP 4 781 983 A1**
(43) Date of publication of application: **29.07.2026**
(21) Application number: 25382040.1
(22) Date of filing: 24.01.2025
(51) Int. Cl.: A61K 31/122, A61P 13/12, C12Q 1/28

(54) **PREVENTION AND/OR TREATMENT OF ACUTE KIDNEY INJURY**

(71) Applicant: Fundación Instituto de Investigación Sanitaria Fundación Jiménez Díaz, 28040 Madrid (ES)
(72) Inventor: GUERRERO MAUVECIN, Juan, 28040 Madrid (ES); SANZ BARTOLOMÉ, Ana Belén, 28040 Madrid (ES); ORTIZ ARDUAN, Alberto, 28040 Madrid (ES); VILLAR GÓMEZ, Natalia, 28040 Madrid (ES)
(74) Representative: Hoffmann Eitle

(57) **Abstract**

The present invention refers to the medical field. Particularly, the present invention refers to a method for the prevention and/or treatment of acute kidney injury.

## Description

### FIELD OF THE INVENTION

The present invention refers to the medical field. Particularly, the present invention refers to a method for the prevention and/or treatment of acute kidney injury (AKI).

### STATE OF THE ART

AKI is a significant clinical condition characterized by the abrupt loss of kidney function, leading to the accumulation of waste products, electrolyte imbalances, and fluid overload.

AKI is often associated with high morbidity and mortality rates, particularly in critically ill patients. The condition may arise due to various causes, including ischemia, nephrotoxins, sepsis, or obstruction, and it is commonly observed in settings such as intensive care units and post-surgical recovery.

Current therapeutic strategies for AKI primarily focus on supportive care, including fluid management, avoidance of nephrotoxic agents, and renal replacement therapy (RRT) in severe cases. Pharmacological approaches have been limited to preventing further kidney damage or managing complications, such as hyperkalemia or acidosis. Despite advancements in medical care, there are no specific pharmacological agents approved for the treatment or prevention of AKI. Research efforts have explored various interventions, including anti-inflammatory agents, antioxidants, growth factors, and inhibitors of apoptosis; however, none have demonstrated consistent efficacy in clinical trials.

The complexity of AKI pathophysiology, involving inflammation, oxidative stress, vascular dysregulation, and tubular injury, poses challenges in identifying effective therapeutic targets.

Moreover, the heterogeneity of patient populations and the multifactorial nature of AKI complicate the development of universal treatment approaches.

The lack of effective pharmacological treatments for AKI represents a critical unmet medical need. AKI contributes significantly to long-term complications, such as the progression to chronic kidney disease (CKD), increased cardiovascular risk, and poor quality of life. Additionally, the condition imposes a substantial economic burden on healthcare systems due to prolonged hospital stays, increased use of RRT, and the need for long-term management of CKD and its associated comorbidities.

New therapeutic strategies are urgently needed to address this unmet medical need. Innovations should aim to target the underlying mechanisms of AKI, such as mitigating oxidative stress, modulating inflammatory responses, and promoting tubular regeneration. A therapeutic approach capable of preventing AKI or expediting recovery could significantly reduce mortality, improve patient outcomes, and lower healthcare costs. The development of such therapies would also have profound implications for high-risk populations, including patients undergoing cardiac surgery, receiving nephrotoxic drugs, or suffering from sepsis.

By addressing these challenges, the present invention seeks to contribute to the advancement of AKI management, offering potential solutions to a pressing clinical problem that remains inadequately addressed by current medical practices.

### DESCRIPTION OF THE INVENTION

### Brief description of the invention

The present invention refers to a method for the prevention and/or treatment of AKI. This invention provides a groundbreaking therapeutic strategy for AKI, addressing a critical unmet medical need. The use of Arachidonate 15-lipoxygenase (ALOX15) inhibitors, particularly Utreloxastat (PTC857), either as standalone agents or in combination therapies, has the potential to significantly improve clinical outcomes and reduce the burden of AKI-related complications.

Particularly, the present invention refers to a method for the prevention and/or treatment of AKI by utilizing inhibitors of enzyme ALOX15, particularly Utreloxastat (PTC857). AKI is a serious medical condition characterized by rapid loss of kidney function, which can lead to severe complications and increased mortality. The invention provides a novel therapeutic approach, leveraging the inhibitory effects of Utreloxastat (PTC857) on ALOX15 activity to mitigate or prevent the underlying pathological processes associated with AKI.

Particularly, in the context of AKI, ferroptosis contributes to tubular epithelial cell damage, inflammation, and loss of renal function. Targeting ferroptosis thus represents a promising therapeutic strategy for preventing and mitigating AKI. This invention introduces a novel strategy to prevent and treat AKI by targeting ferroptosis, leveraging ALOX15 inhibitor Utreloxastat (PTC857) to disrupt the key pathological processes of lipid peroxidation and oxidative stress. The described methods and compositions have the potential to transform AKI management, improving patient outcomes and addressing an urgent unmet medical need.

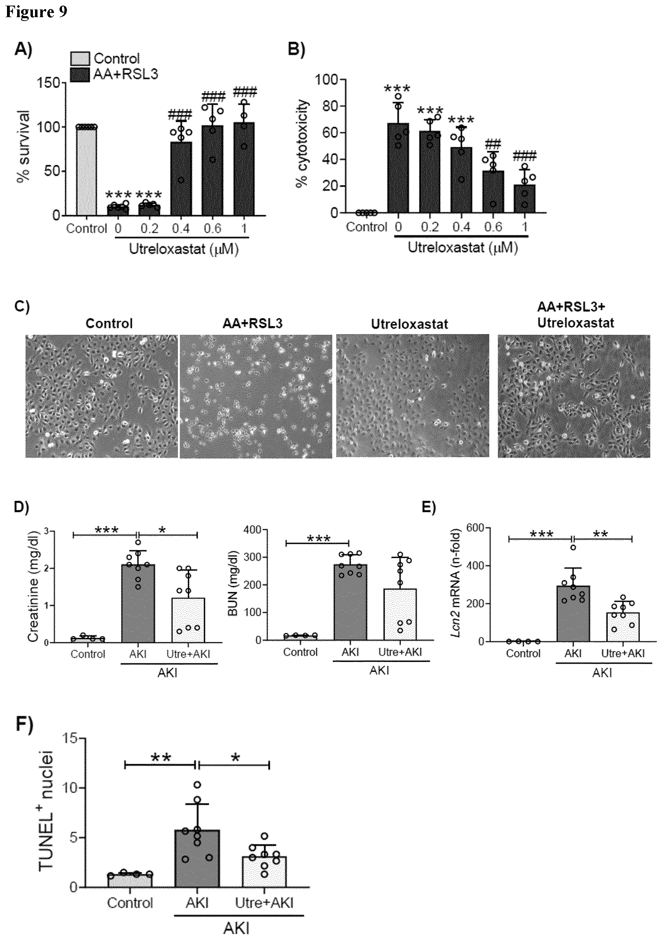

Particularly, the **Example 7** of the present invention shows that Utreloxastat (PTC857) improves cell viability and reduces cell cytotoxicity and detachment (**Figure 9AC**), improves kidney function in folic acid (FA)-induced acute kidney injury (FA-AKI) (**Figure 9D**), reduces tubular injury (**Figure 9E**) and decreases tubule cell death (**Figure 9F**).

So, the first embodiment of the present invention refers to the ALOX15 inhibitor Utreloxastat (PTC857) for use in a method for the prevention and/or treatment of AKI; or to a method for the prevention and/or treatment of AKI which comprises the administration of a therapeutically effective dose or amount of the ALOX15 inhibitor Utreloxastat (PTC857).

The second embodiment of the present invention refers to a pharmaceutical composition comprising the enzyme 15-lipoxygenase inhibitor Utreloxastat (PTC857) and, optionally, pharmaceutically acceptable excipients and/or carriers, for use in a method for the prevention and/or treatment of AKI.

The third embodiment of the present invention refers to an *in vitro* method for screening, identifying and/or producing compounds for use in a method for the prevention and/or treatment of AKI which comprises: a) Assessing ALOX15 activity once the candidate compound has been incubated with ALOX15, and b) wherein if an inhibition of ALOX15 activity is observed, it is indicative that the candidate compound may be effective in a method for the prevention and/or treatment of AKI.

Thus, the pharmaceutical compositions comprising ALOX15 inhibitor Utreloxastat (PTC857), either alone or in combination with other therapeutic agents, is designed for the prevention and/or treatment of AKI. These compositions may include pharmaceutically acceptable carriers and excipients to enhance stability, bioavailability, and patient compliance. Possible formulation examples are as follows:
- Oral tablets containing Utreloxastat (PTC857) combined with excipients like microcrystalline cellulose and magnesium stearate.
- Injectable solutions containing Utreloxastat (PTC857) in saline with stabilizers such as polysorbate 80.

On the other hand, the *in vitro* method for identifying novel ALOX15 inhibitors involves incubating candidate compounds with ALOX15 and measuring enzyme activity. The assay employs techniques such as spectrophotometry or mass spectrometry to detect the formation of lipid peroxides or other ALOX15 reaction products. A possible step-by-step protocol is shown below:
a) Incubate purified ALOX15 enzyme with the candidate compound in a buffered solution.
b) Add a substrate, such as arachidonic acid or linoleic acid.
c) Measure the enzymatic activity using a chromogenic or fluorogenic assay.
d) Identify compounds that significantly reduce enzyme activity compared to controls.

The invention further encompasses combination therapies that pair ALOX15 inhibitor Utreloxastat (PTC857) with other agents, such as:
- Anti-inflammatory drugs (e.g., corticosteroids).
- Antioxidants (e.g., N-acetylcysteine).
- Agents promoting renal perfusion (e.g., vasodilators).

Such combinations are particularly effective in addressing the multifactorial nature of AKI.

The therapeutic methods and compositions described herein are suitable for patients at risk of or suffering from AKI due to conditions such as:
- Ischemia-reperfusion injury (e.g., during cardiac surgery).
- Sepsis or systemic inflammatory response syndrome (SIRS).
- Exposure to nephrotoxic drugs (e.g., aminoglycosides or cisplatin).

For the purpose of the present invention the following terms are defined:
- The term "comprising" means including, but not limited to, whatever follows the word "comprising". Thus, use of the term "comprising" indicates that the listed elements are required or mandatory, but that other elements are optional and may or may not be present.
- The term "consisting of" means including, and limited to, whatever follows the phrase "consisting of'. Thus, the phrase "consisting of" indicates that the listed elements are required or mandatory, and that no other elements may be present.
- By "therapeutically effective dose or amount" of a composition is intended an amount that, when administered as described herein, brings about a positive therapeutic response in a subject having AKI. The exact amount required will vary from subject to subject, depending on the age, and general condition of the subject, the severity of the condition being treated, mode of administration, and the like. An appropriate "effective" amount in any individual case may be determined by one of ordinary skill in the art using routine experimentation, based upon the information provided herein.

### Description of the figures

**Figure 1****.** ACSL4 is upregulated during AKI induced by folic acid overdose.
**Figure 2****.** RSL3, inductors of ferroptosis, cause cell death in HK2 tubular cells cultured with arachidonic acid (AA).
**Figure 3****.** Fer-1 and Lpx-1, two inhibitors of ferroptosis, prevent AA+RSL3-induced cell death in HK2 tubular cells.
**Figure 4****.** Silencing of ACSL4, key enzyme on ferroptosis pathway, prevents AA+RSL3-induced cell death.
**Figure 5****.** Pharmacological inhibition of ACSL4 with troglitazone prevents cell death and lipid peroxidation in AA+RSL3-stimulated tubular cells.
**Figure 6****.** ALOX15 is upregulated in FA-AKI and an inhibitor of ALOX15, PD146176, prevents ferroptosis in HK2 tubular cells induced by AA+RSL3.
**Figure 7****.** Oxide-lipidomic analysis of AA+RSL3-stimulated HK2 cells reveals lipid peroxidized species that are key hallmarks of ferroptosis and are decreased by inhibition of ACSL4 or Alox15.
**Figure 8****.** Inhibition of ACSL4 with troglitazone reduces tubular damage, parenchymal cell death, lipid peroxidation and renal dysfunction in AKI induced by folic acid overdose.
**Figure 9****.** Pre-treatment with Utreloxastat prevents cell death induced by AA+RSL3 in HK2 cells, and reduces kidney dysfunction, parenchymal cell death and tubular cell damage in AKI induced by folic acid overdose.

### Detailed description of the invention

The present invention is illustrated by means of the Examples set below without the intention of limiting its scope of protection.

### Example 1. ACSL4 expression is upregulated in folic acid (FA)-induced acute kidney injury (FA-AKI)

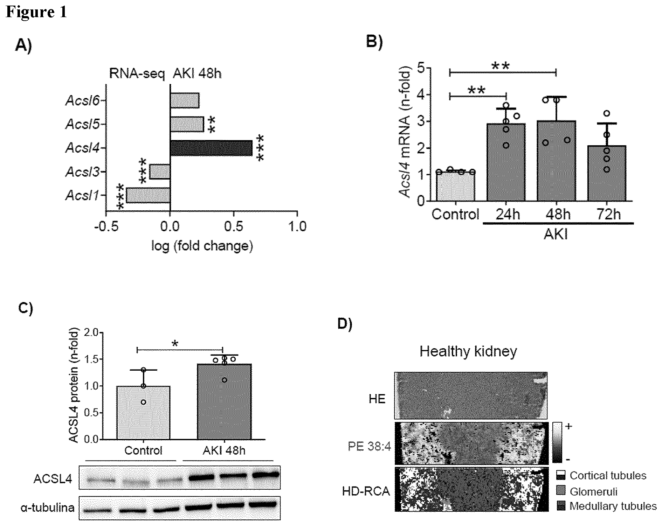

ACSL4 catalyzes the esterification of arachidonic acid (AA) to form AA phospholipids (AA-PL), which are the fuel for ferroptosis; indeed, oxidized AA-PL species have been described as ferroptotic hallmarks in model of folic acid-AKI. Kidney RNA-seq of FA-AKI at 48h hours identified *Acsl4* as the most upregulated gene between members of Acsl family (**Figure 1A**). In FA-AKI, increased kidney *Acsl4* mRNA levels were already evident at 6 hours, further increased at 48 hours and persisted increased for 72 hours post-injury as was assessed by qRT-PCR (**Figure 1B**). Total ACSL4 protein was also increased in FA-AKI at 48 hours (**Figure 1****.C**). Indeed, we have observed by MALDI-IMS that in healthy mice, the kidney cortex showed a higher relative abundance on AA-PE, the main PLs oxidized during ferroptosis analyzed (**Figure 1D**). This higher PE content in the kidney, along with a higher expression of ACSL4 observed during AKI, could derive in an increased AA-PE and a higher susceptibility to ferroptosis, which prompts the interest on this enzyme as ferroptotic target in FA-AKI.

### Example 2. High arachidonic acid content sensitizes renal tubular cells to ferroptosis

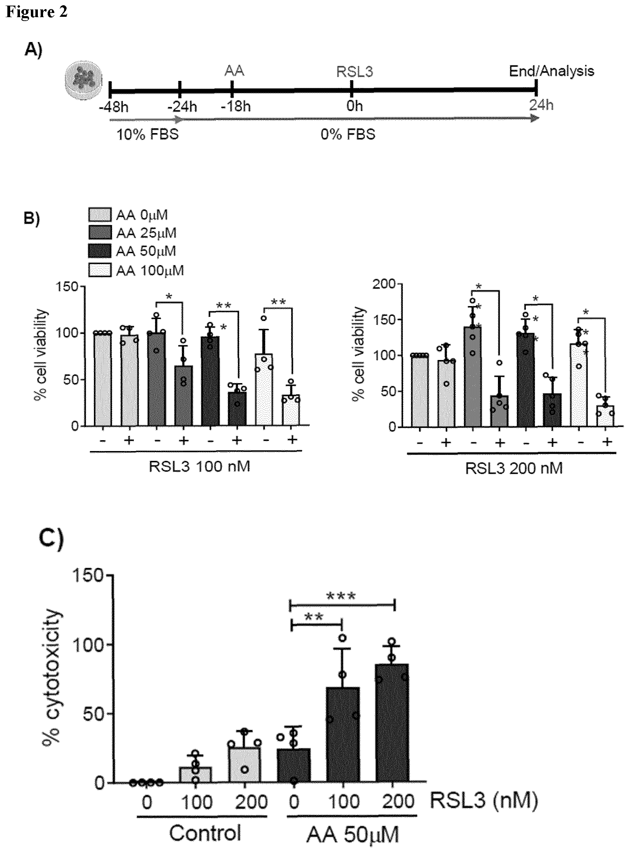

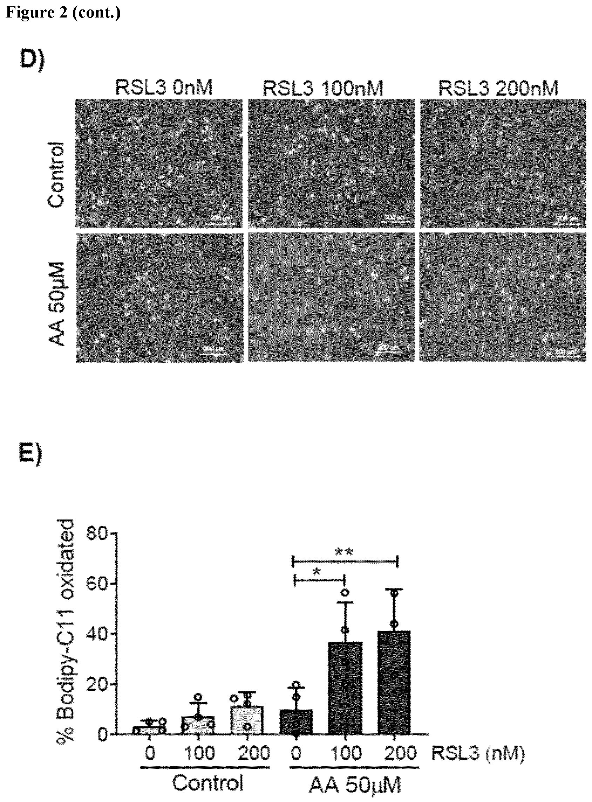

Variable susceptibility to ferroptosis among different cell types and physiological contexts may depend on different lipid profiles and the strength of cellular antioxidant defence. To explore the potential of ACSL4-mediated activation of polyunsaturated fatty acids (PUFA) as the instigator of ferroptosis in the kidney, we exposed human renal proximal tubular HK2 cells with its preferred substrate, arachidonic acid (AA), under sublethal RSL3 conditions (**Figure 2A**).

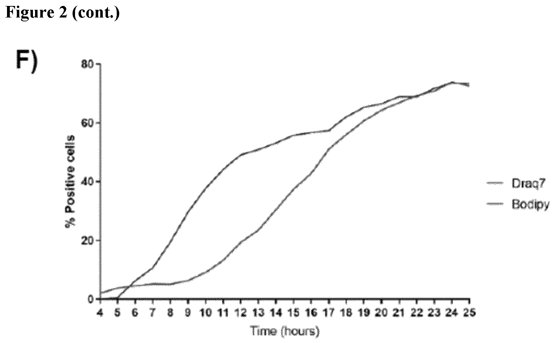

Neither AA nor RSL3 alone caused cell death, while co-incubation of both resulted in reduced cell viability and this effect was dependent of AA and RSL3 doses **(****Figure 2B****).** Moreover, the combination of AA and RSL3 promotes cellular lysis and cell detachment as was assessed by a LDH release assay and phase contrast images respectively **(Figure 2CD****).** Additionally, we confirmed a higher lipid peroxidation rate under these conditions by flow cytometry analysis of Bodipy⁺ cells **(****Figure 2E****)**, and by confocal microscopy in live cells we observed that lipid peroxidation occurs before cell lysis **(****Figure 2F****).**

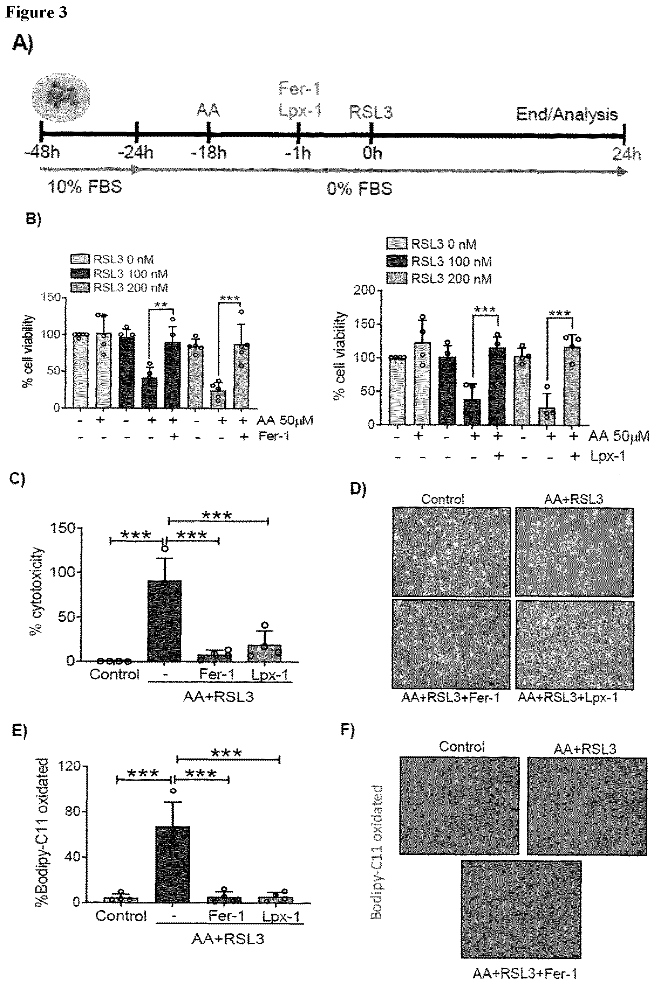

Next, to confirm that AA+RSL3 combination induces cell death by ferroptosis we pretreated cells with ferrostatin-1 (Fer-1) or liproxstatin-1 (Lpx-1), two specific ferroptosis inhibitors **(****Figure 3A****)**. Both inhibitors prevented the loss of cell viability, cell lysis and cell detachment, even a higher dose of RSL3 **(****Figure 3B-D****).** Moreover, the pretreatment with either inhibitor reduced lipid peroxidation promoted by AA+RSL3 **(Figure 3EF),** which confirms ferroptosis as the driving mechanism of cell death.

### Example 3. Targeting ACSL4 prevents ferroptosis induced by high PUFA content in tubular cells

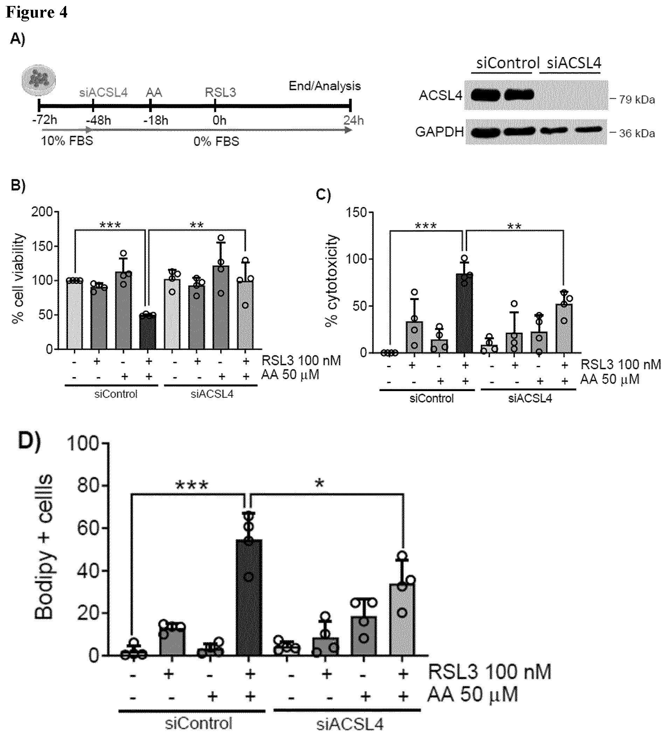

To test the relevance of ACSL4 on tubular ferroptosis, we transfected HK2 cells with specific siRNAs targeting *Acsl4* mRNA for transcriptional silencing prior to ferroptosis induction **(****Figure 4A****).** We observed that impaired expression of ACSL4 significantly reduces cell death and lipid peroxidation **(****Figure 4B-D****).**

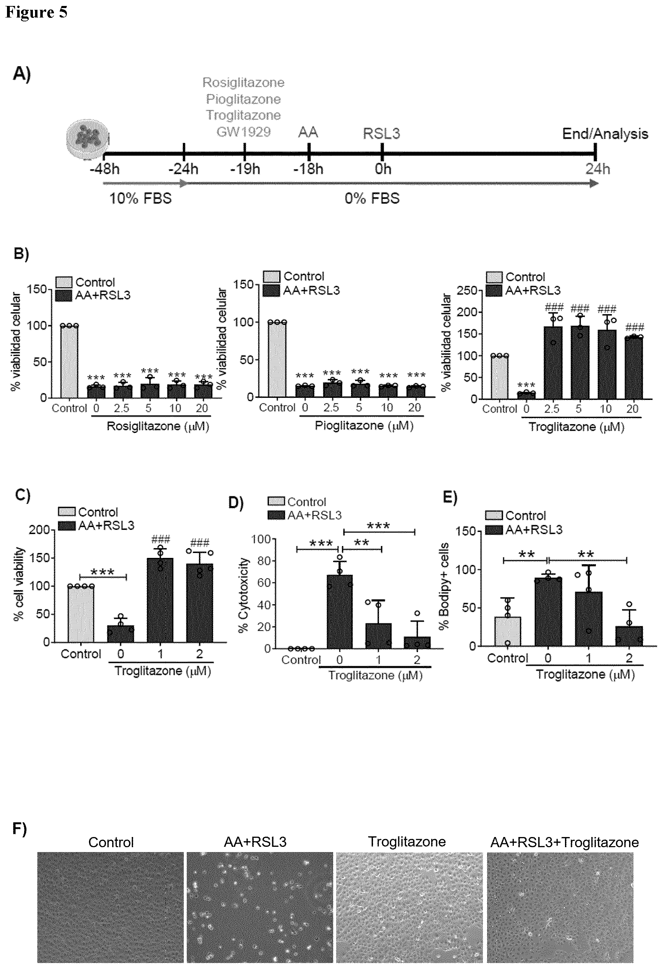

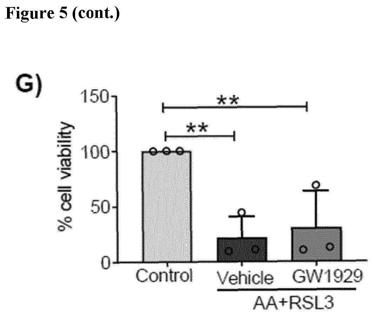

Additionally, we targeted ACSL4 pharmacologically with the thiazolidinedione (TZD) family members Rosiglitazone, Troglitazone and Pioglitazone (**Figure 5A**). According to previous studies, these PPARγ agonists, commonly used for insulin sensitization in clinical settings, have demonstrated to selectively inhibit ACSL4 and other isoforms. Our results indicate that only Troglitazone offered protection against cell death and lipid peroxidation in HK2 cells treated with AA+RSL3 (**Figure 5B**). Interestingly, our finding aligns with prior reports pointing out Troglitazone as the most anti-ferroptotic TZD over other family members. Indeed, low dose of Troglitazone increased cell viability, and reduced cytotoxicity, lipid peroxidation and cell detachment **(****Figure 5C-F****).** To discard possible off-target effects over PPARγ gene transcription, we used GW1929, a non-TZD PPARγ agonist, and confirmed the absence of protective effects (**Figure 5G****).**

The chemical structure of Troglitazone includes a chromanol ring, suggesting antioxidant properties like tocopherols. However, this protection, which is independent of ACSL4 inhibition, appears to be partial, as the protection offered by troglitazone is greater than that offered by tocopherol.

### Example 4. ALOX15 is upregulated in FA-AKI and mediates ferroptosis in cultured tubular cells

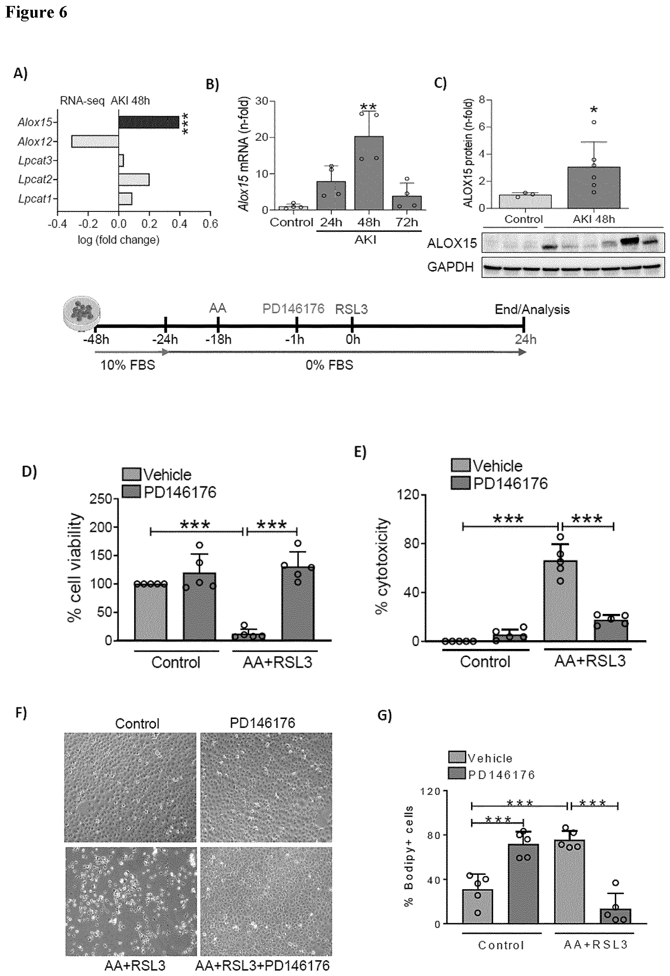

ALOX15 belong to Lipoxygenase (LOXs) family, and it uses AA as substrate to generate 15-hydroxyeicosatetraenoic (HpETE). We have observed that *Alox15* appeared as a significantly overexpressed gene in a kidney RNA-seq of FA-AKI at 48h, while other genes associated with lipid metabolism and ferroptosis are downregulated, as Alox12, or show weak changes as Lpcat 1/2/3 (**Figure 6A**). Additionally, the upregulation of ALOX15 in FA-AKI was confirmed at mRNA and protein level by RT-PCR and western blot respectively **(Figure 6BC).**

To evaluate the potential of ALOX15 as a mediator of tubular ferroptosis, we pretreated HK2 tubular cells with PD146176, an inhibitor of ALOX15, one hour before to add ferroptosis inductor AA+RSL3. These experiments showed that inhibition of ALOX15 improved cell viability, and reduced cell cytotoxicity, cell detachment and lipid peroxidation induced by AA+RSL3 **(****Figure 6D-G****).**

### Example 5. ACSL4 and ALOX15 inhibition prevented the generation of oxidized phospholipids characteristics of ferroptosis in cultures tubular cells

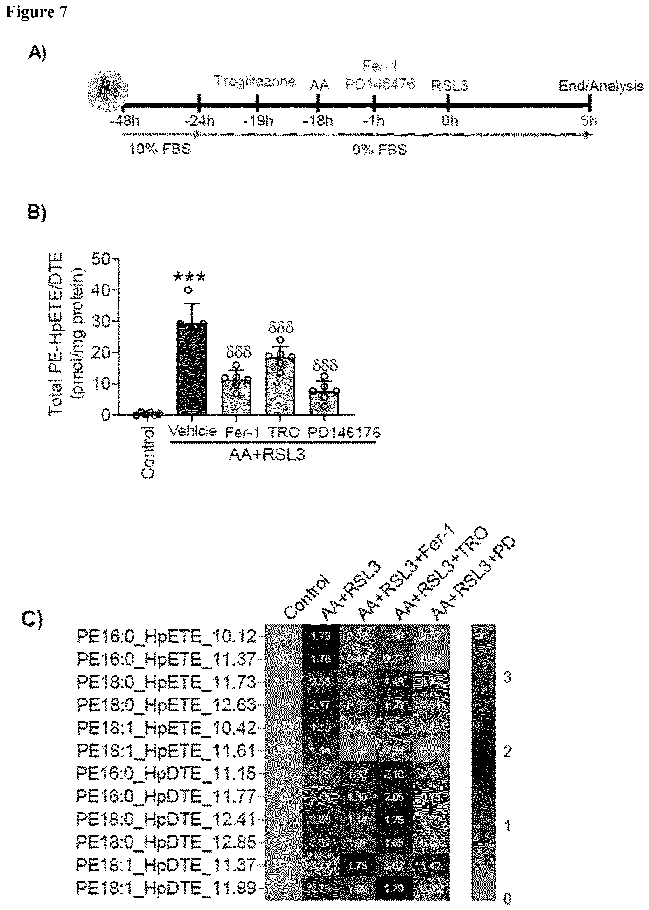

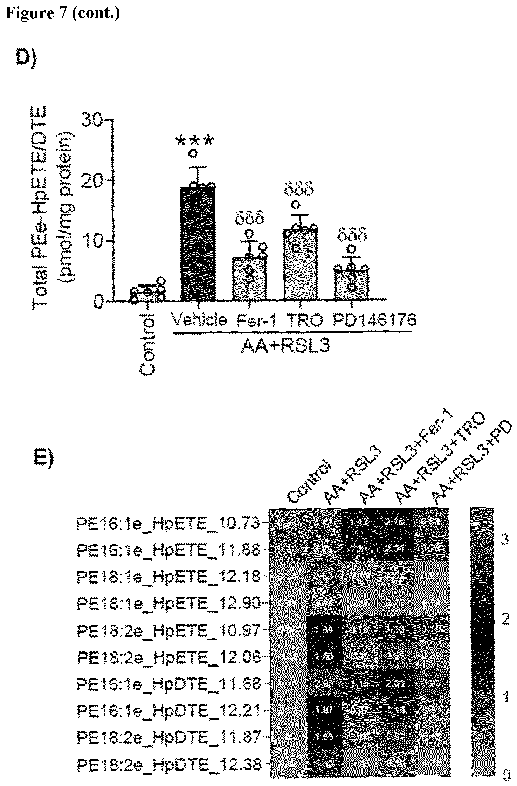

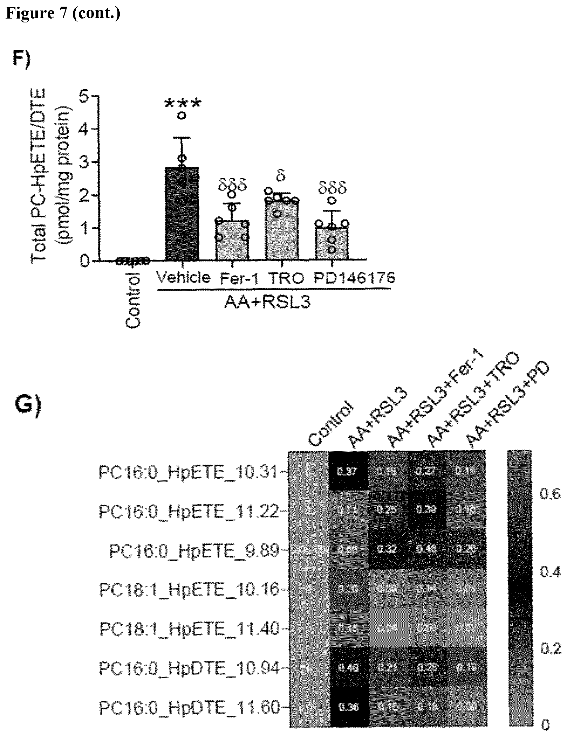

The oxydolipidome of HK2 cells treated with ferroptosis inductor AA+RSL3 for 6 hours was analyzed by mass spectrometry **(****Figure 7A****).** We found that AA+ RSL3 stimulation led to the generation of oxygenated PLs containing HpETE and hydroxydocosatetraenoico (Hp-DTE), which are derivatives of AA (20:4) and adrenic acid (22:4) respectively **(****Figure 7B-G****).** However, free HpETE and HpDTE were not detected, this is according with ferroptosis induction since mainly esterified PUFAs are oxidized.

We detected three classes of oxygenated PLs, being phosphatidylethanolamines (PE-HpETE/DTE) the most abundant class **(****Figure 7B****)** following by ether-PEs (PEe-HpETE/DTE) **(****Figure 7D****),** and in a minus amount phosphatidylcholines (PC-HpETE/DTE) **(****Figure 7F****).** The pretreatment with ferroptosis inhibitor Fer-1, ACSL4 inhibitor Troglitazone and ALOX15 inhibitor PD146176 prevented the increase of these oxidized classes **(****Figure 7B****, D, F),** confirming that ferroptosis, ACSL4 and ALOX15 contribute to lipid oxidation.

The oxidized PE-HpETE and PE-HpDTE species detected (**Figure 7C**) are associated to ACSL4 and ferroptosis, and their levels were also increased in kidneys of mice with Folic acid-AKI where ferroptosis contribute to kidney failure. Now, we showed that the inhibition of ferroptosis, ACSL4 or ALOX15 reduced the levels of these species, confirming that they are a hallmark of ferroptosis (**Figure 7BC**). The PEe-HpETE/HpDTE (plasmalogens) are related with ferroptosis, and we have observed that treatment with ferroptosis inhibitors also reduced their levels (**Figure 7DE**). PC-HpETE/HpDTE species increased a lower level the PE species, but they are also dependent on ferroptosis, ACSL4 and ALOX15 (**Figure 7FG**).

### Example 6. Protective effect of Troglitazone in folic acid-AKI

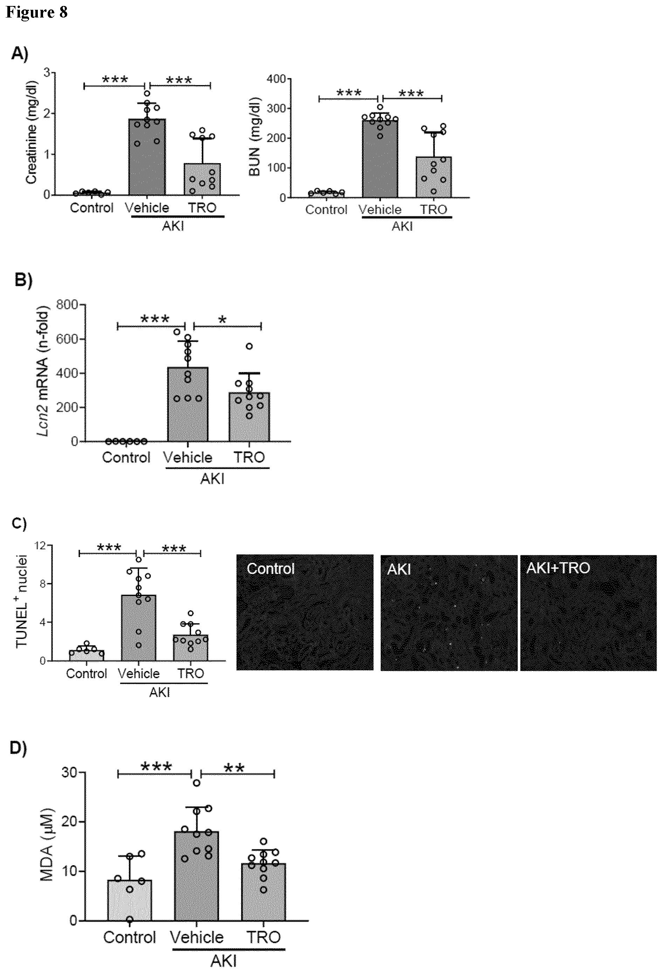

Folic acid-AKI is a model that reproduces the hallmark of clinical AKI such as tubular lesion and kidney failure, and it was reported that ferroptosis of tubular cells contribute to first injury in this model. In base of our *in vitro* results, we explored the therapeutic potential of Troglitazone injection in this model. We observed that Toglitazone pre-treatment improved kidney function assessed by serum creatinine and urea levels (**Figure 8A**) and reduced tubular injury measured by *Lcn2* gene expression (**Figure 8B**). The treatment with Troglitazone also reduces the level of cell death, measured by TUNEL staining (**Figure 8C**), and lipid peroxidation, measured by kidney MDA levels (**Figure 8D**). These results suggest that Troglitazone has a protective effect in FA-AKI models, targeting the activation of ferroptosis.

### Example 7. Comparative assay between different ALOX15 inhibitors. Utreloxastat (PTC857) targeting of ALOX15 ameliorate FA-AKI by targeting ferroptosis.

To explore the role of ALOX15 *in vivo,* we pretreated the mice with the inhibitor PD146476, but it did not work *in vivo.* Thus, we evaluated the effect of Utreloxastat (PTC857), which has been described as an ALOX15 inhibitor that is undergoing a safety and efficacy clinical trial in amyotrophic lateral sclerosis (NCT05349721). First, we explored the effect of Utreloxastat in HK2 cells exposed to AA+RSL3, observing that it improved cell viability and reduced cell cytotoxicity and detachment **(****Figure 9A-C****)**. Next, we evaluated whether ALOX15 targeting with Utreloxastat prevents AKI *in vivo.* Preventive Utreloxastat improved kidney function in FA-AKI (**Figure 9D**), reduced tubular injury (**Figure 9E**) and decreased tubular cell death (**Figure 9F**).

## Claims

1. Enzyme 15-lipoxygenase inhibitor Utreloxastat (PTC857) for use in a method for the prevention and/or treatment of acute kidney injury.

2. Pharmaceutical composition comprising the enzyme 15-lipoxygenase inhibitor Utreloxastat (PTC857) and, optionally, pharmaceutically acceptable excipients and/or carriers, for use in a method for the prevention and/or treatment of acute kidney injury.

3. *In vitro* method for screening, identifying and/or producing compounds for use in a method for the prevention and/or treatment of acute kidney injury which comprises: a) Assessing 15-lipoxygenase enzyme activity once the candidate compound has been incubated with 15-lipoxygenase enzyme, and b) wherein if an inhibition of enzyme 15-lipoxygenase activity is observed, it is indicative that the candidate compound may be effective in a method for the prevention and/or treatment of acute kidney injury.
